Europäisches Patentamt

(19) European Patent Office

Office européen des brevets

(11) Publication number: **0 011 350**
A1

## (12) EUROPEAN PATENT APPLICATION

(21) Application number: 79200672.8

(22) Date of filing: 15.11.79

(51) Int. Cl.³: **C 12 P 19/14**
 C 13 K 11/00

(30) Priority: 18.11.78 NL 7811389

(43) Date of publication of application:
28.05.80 Bulletin 80 11

(84) Designated Contracting States:
AT BE DE FR GB IT NL SE

(71) Applicant: STAMICARBON B.V.
Postbus 10
NL-6160 MC Geleen(NL)

(72) Inventor: Kerkhoffs, Pieter Laurens
Chopinstraat 1
NL-6164 CE Geleen(NL)

(74) Representative: Van de Panne, Vitus Nicolaas et al,
Octrooibureau DSM Postbus 9
NL-6160 MA Geleen(NL)

(54) Preparation of fructose.

(57) The invention concerns a process for the preparation of fructose by hydrolysis of fructose polymers (inulin or levan) from vegetable origin.

According to the invention, comminuted vegetable matter containing a fructose polymer is brought in contact in an aqueous medium with an enzyme that hydrolyses fructose polymers, and fructose is isolated from the solution thus obtained. Specifically, inulin containing matter is treated with the enzyme inulase.

An advantage of the process is, that the formation of byproducts can be avoided, resulting in easy recovery of the fructose in crystalline form.

EP 0 011 350 A1

Croydon Printing Company Ltd.

STAMICARBON B.V.

1                                                   3039

## PREPARATION OF FRUCTOSE

.Fructose is a natural compound with a greater sweetening power than natural sugar, and is better tolerated by diabetics.

Various fructose polymers occur in nature, notably inulin and levan. Inulin is contained in various plants, mainly in tubers and roots of plants belonging to the family of the composites. Levan occurs i.a. in grasses and grains and as a product of bacterial polymerization.

The recovery of fructose from inulin by chemical or enzymatic hydrolysis is a known process. Inulin itself is recovered from parts of plants by extraction.

The object of the invention is a simpler process for the preparation of fructose from plants containing a fructose polymer. According to the invention fructose is obtained by placing comminuted vegetable matter containing a fructose polymer in contact, in an aqueous medium, with an enzyme that hydrolyses fructose polymers and, after removal of the remaining vegetable matter, isolating in a known way, the fructose obtained by enzymatic conversion.

The advantage of the process according to the invention is that the extraction step is avoided and that a pure fructose is obtained that can be crystallized more readily than the fructose obtained by chemical hydrolysis. An important advantage is that the process according to the invention can be effected in a very weakly acid to neutral medium. As a result, the formation of by-products, such as the acid-catalyzed formation of fructose dianhydride and the base-catalyzed aldol condensation, is suppressed almost completely. In the usual acid inulin extraction and inulin hydrolysis significant

2

amounts of by-product do form.

The recovery of fructose from material containing levan is effected by means of the enzyme levanase and its recovery from material containing inulin by means of inulase.

The starting material used is preferably one containing inulin, as inulin is contained in considerable amounts in the tubers or roots of plants that are easy to grow. In many cases the inulin content ranges between 10 and 20 % by weight. Some suitable vegetable materials are dahlia tubers, Helianthus Tuberosus, members of the genus Cichoreum, scorzonera, elecampane (Inula Helenium), costus roots (Saussurea lappa) and Jerusalem artichokes.

The parts of the plants rich in inulin are comminuted to a pulpy mass. The means known in the sugar and starch industries may be used for this purpose, such as graters, choppers and ball mills.

Inulase is the trivial name of $\beta(1-2)$ fructan-fructano-hydrolase, enzyme number EC 3.2.1.7. It is a well-known enzyme capable of breaking $\beta(1-2)$ fructose bonds. Inulase can be obtained from vegetable material, e.g. from Jerusalem artichokes, but is obtained in practice from cultures of micro-organisms, such as, i.a., Saccharomyces fragilis, Aspergillus niger, and Helminthosporium oryzae. The enzyme may be used in the crude or pure form, or as a cellular mass with inulase activity. Here, the activity of the preparations will be expressed in standard units (SU), one SU being the activity required to produce 1 micromole of fructose per minute from inulin. The amount of enzyme preparation used depends, i.a., on the inulin content of the vegetable matter, the activity of the preparation, and the time available for the reaction. In most cases an amount of enzyme preparation corresponding to an activity of between 10 and 6000 SU, more in particular between 100 and 3000 SU, will be used per gram of inulin contained in the vegetable matter.

The temperature and pH in the reaction are preferably those at which the enzyme has optimum activity. What values are chosen consequently depends on the origin of the enzyme preparation. The pH will generally range between 3.8 and 8.5, more in particular between 4.5 and 6.5. In the latter range the formation of by-products is negligible. The temperature will generally be between 10 and 90 $^o$C, more in particular between 40 and 75 $^o$C.
The temperature may be varied during the reaction.

The reaction is effected in an aqueous medium by mixing the vegetable pulp and the enzyme preparation, optionally with addition of more water. Also, pH regulators and enzyme activators may be added. The concentration of the reaction mixture will generally be between 10 and 50 % by weight of solids. Optionally the enzyme preparation may be added in portions as the reaction proceeds. The reaction may be carried out in a single stirred reactor or in a series of stirred reactors. In most cases the residence time will be between 1 and 24 hours.

Upon termination of the reaction, the remaining vegetable matter is separated out by filtration or centrifugation. The aqueous solution of fructose can be processed further in the usual way to purify and recover the fructose. Residual enzyme can be removed by ultrafiltration or treatment with an ion exchanger. The fructose obtained according to the invention is found to crystallize better than the fructose subjected to dilute-acid extraction and hydrolysis.

The invention will be elucidated in the following examples, but without being restricted to this mode of realization.

## Example I

Chicory roots were washed, chopped, freeze-dried and ground. Freeze-drying was effected to enable the material to be stored for several months without degradation, and can be omitted in practice. 35 g of the powder obtained in this way was suspended in 300 g of water. 50 g of an aqueous inulase solution was added to this suspension, after which the pH of the mixture was adjusted to 5.0 by means of acetic acid and the mixture was heated at 50 $^o$C for 24 hours while being stirred. The inulase used was a crude preparation of unknown activity.

After 24 hours the suspension was centrifuged. This left 5 g of solid matter. The solution remaining after centrifugation

-4-

was stirred with 25 g of activated carbon for 0.5 hour. After the carbon had been removed by filtration, the solution was passed first across a strongly acid ion exchanger (175 ml of Amberlyst-15, of Rohm & Haas) and then across a weakly basic ion exchanger (175 ml of Duolite A-7, of Dow Chemical). The decolourized neutral solution was then subjected to evaporation. After addition of seed crystals and crystallization, the resulting solid substance was stirred with 100 cc of ethanol at 5 °C. The crystal mass was recovered by filtration, washed with cold ethanol, and dried. Thus 24.5 grams of fructose were obtained. No more inulin or fructose could be extracted from the remaining vegetable matter by means of boiling water or dilute hydrochloric acid. This means that the fructose is recovered virtually quantitatively, allowance being made for the losses during processing.

Example II

Ten grams of freeze-dried ground chicory roots with a water content of 1.3 % by weight was stirred with 90 ml of water and 6 g of aqueous inulase solution for 20 hours at a temperature of 55 °C and at a pH of 5.0 adjusted by means of acetic acid. The inulase solution had an activity of 300 SU per gram.

1.5 g of residual material was removed by centrifugation. Analysis showed the solution thus obtained to contain 7.15 g of fructose and 0.90 g of glucose. The amount of glucose is higher than would be calculated from the hydrolysis of inulin. An explanation is that the enzyme preparation also possesses some invertase activity, so that hydrolysis of the sucrose probably also contained in the roots can occur. However, the small amount of glucose can easily be separated off, as it remains in the mother liquor upon crystallization.

Pure crystalline fructose could be recovered from the sugar solution in the way described in Example I.

A blank was run by repeating the above procedure, with the sole difference that 6 g of water was used instead of the

phase solution. The aqueous phase obtained upon centrifugation contained a negligibly small amount of fructose that could not be determined quantitatively. This shows that the amount of inulase that may be contained in the chicory roots can be neglected, and the addition of inulase according to the invention is essential.

Example III

Freeze-dried ground roots of belgian endive (25 g) with a water content of 1.5 % by weight were treated as in example I with 15 g of an aqueous inulase solution (300 SU/g), yielding 3.8 g of residue and an aqueous solution from which 16.8 g of crystalline fructose could be obtained by the process described in example I.

Example IV

Freeze-dried ground dahlia tubers (20 g) with a water content of 1.6 % by weight were treated as in example I with 13.4 g of aqueous inulase solution (300 SU/g) and 200 ml of water, yielding 15.1 g of crystalline fructose.

6           3039

# C L A I M S

1. Process for the preparation of fructose from vegetable matter, characterized in that comminuted vegetable matter containing a fructose polymer is contacted, in an aqueous medium, with an enzyme that hydrolyzes fructose polymer and, after removal of the remaining vegetable matter, the fructose obtained by enzymatic conversion is isolated in a usual way.

2. Process according to claim 1, characterized in that vegetable matter containing inulin is contacted with inulase in an aqueous medium.

3. Process according to claims 1-2, characterized in that the reaction is effected at a pH of between 3.0 and 8.5.

4. Process according to claim 3, characterized in that the reaction is effected at a pH of between 4.5 and 6.5.

5. Process according to claims 1-4, characterized in that the reaction is effected at a temperature of between 40 and 75 $^{o}$C.

6. Process according to claims 2-5, characterized in that the amount of enzyme preparation with inulase activity used per gram of inuline contained in the vegetable matter corresponds to an inulase activity of between 10 and 6000 SU.

7. Process according to claim 6, characterized in that the amount of enzyme preparation used corresponds to an activity of between 100 and 3000 SU.

8. Process for the preparation of fructose from vegetable matter according to the invention, as described in the text and elucidated in the examples.

9. Fructose obtained by the process according to any one or more of the claims 1-8.

0011350

European Patent Office

**EUROPEAN SEARCH REPORT**

Application number

EP 79 20 0672

| Category | Citation of document with indication, where appropriate, of relevant passages | Relevant to claim |
|---|---|---|
| | CHEMICAL ABSTRACTS, vol. 88, no. 23, 5. June 1978, abstract nr. 168490a, Columbus, Ohio, US, <br><br> & JP - A - 77 136 928 (SANKYO CO LTD) <br><br> * The whole abstract * <br><br> -- | 1-9 |
| | CHEMICAL ABSTRACTS, vol. 88, nr. 23, 5. June 1978, abstract nr. 168491b, Columbus, Ohio, US, <br><br> & JP - A - 77 136 929 (SANKYO CO LTD) <br><br> * The whole abstract * <br><br> -- | 1-9 |
| | CHEMICAL ABSTRACTS, vol. 57, no. 6, 17. September 1962, abstract nr. 7589b, Columbus, Ohio, US, M. WARTERESIEWICZ.: "Hydrolysis of glucofructosans of topinambou" <br><br> & Ann.Univ.Mariae Curie-Sklodowska, Lublin-Polonia, 163-88 (1961) <br><br> * The whole abstract * <br><br> -- | 1-9 |
| | CHEMICAL ABSTRACTS, vol. 44, no. 6, 25. March 1950, abstract nr. 2778g, Columbus, Ohio, US, S. INAGAKI et al.: "Preparation of fructose from Jerusalem artichoke" <br><br> & J.Pharm.Soc.Japan, 69, 418-24 (1949) <br><br> ./. | 1-9 |

**DOCUMENTS CONSIDERED TO BE RELEVANT**

CLASSIFICATION OF THE APPLICATION (Int. Cl.³)

C 12 P 19/14
C 13 K 11/00

TECHNICAL FIELDS SEARCHED (Int.Cl.³)

C 13 K 11/00
C 12 P 19/14
C 12 D 13/10

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | | |
|---|---|---|
| The present search report has been drawn up for all claims | | |
| Place of search <br> The Hague | Date of completion of the search <br> 22-02-1980 | Examiner <br> LENSEN |

EPO Form 1503.1  06.78

| | **DOCUMENTS CONSIDERED TO BE RELEVANT** | | CLASSIFICATION OF THE APPLICATION (Int. Cl. 3) |
|---|---|---|---|
| Category | Citation of document with indication, where appropriate, of relevant passages | Relevant to claim | |
| | x The whole abstract x<br><br>--<br><br>DE - B - 1 042 364 (KEIMDIAET)<br>x Column 2, lines 25-34; claims x<br><br>---- | 1-9 | |
| | | | TECHNICAL FIELDS SEARCHED (Int. Cl. 3) |

European Patent Office

**EUROPEAN SEARCH REPORT**

EPO Form 1503.2   06.78